# EUROPEAN PATENT APPLICATION

(11) **EP 4 756 016 A1**
(43) Date of publication of application: **10.06.2026**
(21) Application number: 24848040.2
(22) Date of filing: 19.07.2024
(51) Int. Cl.: C12N 9/02, C12N 15/53, C12Q 1/66, G01N 21/76

(54) **MODIFIED FIREFLY LUCIFERASE, PREPARATION METHOD THEREFOR AND USE THEREOF**

(30) Priority: 01.08.2023 CN 202310958770
(71) Applicant: Beyotime Biotech Inc, Shanghai 201611 (CN)
(72) Inventor: LI, Shengjian, Shanghai 201611 (CN); ZHAO, Xinyan, Shanghai 201611 (CN); TANG, Yannan, Shanghai 201611 (CN)
(74) Representative: Hoffmann Eitle
(86) International application number: PCT/CN2024/106287
(87) International publication number: WO 2025/026081

(57) **Abstract**

Provided in the present invention are a series of substrate binding pocket-modified firefly luciferases having high enzyme activity and high thermal stability, a preparation method therefor and the use thereof. Through the analysis of the spatial structure of the substrate binding pocket of the firefly luciferase, and on the basis of considering a large number of amino acid sites related to enzyme activity and thermal stability of firefly luciferase, the present inventors design, construct and detect a series of firefly luciferase mutants. The firefly luciferase has a high enzyme activity and thermal stability.

## Description

The disclosure claims priority to the patent application with application number CN 202310958770.6, submitted on August 1, 2023; the entire contents of which are hereby incorporated by reference.

### TECHNICAL FIELD

The disclosure belongs to the field of biotechnology; more specifically, it relates to a modified firefly luciferase, a preparation method therefor and a use thereof; the firefly luciferase exhibits high enzyme activity and thermal stability.

### BACKGROUND OF THE DISCLOSURE

Firefly luciferase catalyzes the oxidation of firefly luciferin in an aerobic environment, accompanied by bioluminescence. This reaction requires magnesium ions and ATP. In the presence of excess firefly luciferase and firefly luciferin, the intensity of the fluorescence generated by the reaction is directly proportional to the amount of ATP. Therefore, firefly luciferase is widely used in the field of biotechnology for ATP detection. In previous researches, the inventor introduced mutation(s) into wild-type firefly luciferase for improving enzyme activity and thermal stability of the luciferase.

With technological advancements, higher requirements have been raised in this field for cell viability detection and other uses. To better utilize this technology, there is an urgent need to further enhance the enzyme activity and thermal stability of firefly luciferase.

### SUMMARY OF THE DISCLOSURE

The purpose of the present disclosure is to provide a series of substrate-binding pocket modified-firefly luciferase mutants, a preparation method therefor and a use thereof; the firefly luciferase exhibits high enzyme activity and thermal stability.

In the first aspect, the present disclosure provides a method for enhancing enzyme activity and/or thermal stability of a firefly luciferase, comprising: mutating and modifying the firefly luciferase to form a firefly luciferase mutant, wherein the mutant has a spatial structure adjacent to the substrate-binding pocket, contributing to the recognition and binding of the substrate or the stability of the protein's spatial conformation.

In another aspect, the present disclosure provides a substrate-binding pocket-modified firefly luciferase mutant, wherein the mutant has a spatial structure adjacent to the substrate-binding pocket, contributing to the recognition and binding of the substrate or the stability of the protein's spatial conformation, and the mutant is a protein comprising the following mutation(s) (comprising combinations of mutation(s)) corresponding to the amino acid sequence shown in SEQ ID NO: 2:
A. mutation(s) selected from (a)-(t) or a combination thereof:
   (a) a mutation of the amino acid F at position 293 to L (M1);
   (b) a mutation of the amino acid Y at position 254 to S (M2);
   (c) a mutation of the amino acid V at position 239 to A (M5);
   (d) a mutation of the amino acid N at position 137 to K, a mutation of the amino acid A at position 235 to T, a mutation of the amino acid K at position 280 to R, a mutation of the amino acid N at position 333 to D, and a mutation of the amino acid I at position 422 to V (M13);
   (e) a mutation of the amino acid K at position 57 to R (M14);
   (f) a mutation of the amino acid T at position 42 to A, a mutation of the amino acid E at position 282 to D, a mutation of the amino acid K at position 392 to I, and a mutation of the amino acid D at position 426 to V (M15);
   (g) a mutation of the amino acid S at position 525 to P (M17);
   (h) a mutation of the amino acid N at position 231 to A, R or K (M20, M21, or M22);
   (i) a mutation of the amino acid A at position 229 to R or K (M23 or M24);
   (j) a mutation of the amino acid A at position 229 to K and a mutation of the amino acid N at position 231 to K (M25);
   (k) a mutation of the amino acid N at position 231 to K and a mutation of the amino acid F at position 293 to L (M27);
   (l) a mutation of the amino acid A at position 229 to R and a mutation of the amino acid N at position 231 to R (M29);
   (m) a mutation of the amino acid N at position 231 to R and a mutation of the amino acid F at position 293 to L (M30);
   (n) a mutation of the amino acid A at position 229 to K and a mutation of the amino acid N at position 231 to A (M31);
   (o) a mutation of the amino acid N at position 231 to A and a mutation of the amino acid F at position 293 to L (M33);
   (p) a mutation of the amino acid A at position 229 to K, a mutation of the amino acid N at position 231 to K, and a mutation of the amino acid F at position 293 to L (M34);
   (q) a mutation of the amino acid A at position 229 to R, a mutation of the amino acid N at position 231 to K, and a mutation of the amino acid F at position 293 to L (M35);
   (r) a mutation of the amino acid A at position 229 to K, a mutation of the amino acid N at position 231 to R, and a mutation of the amino acid F at position 293 to L (M36);
   (s) a mutation of the amino acid A at position 229 to R, a mutation of the amino acid N at position 231 to R, and a mutation of the amino acid F at position 293 to L (M37);
   (t) a mutation of the amino acid A at position 229 to K, a mutation of the amino acid N at position 231 to K, and a mutation of the amino acid F at position 293 to L (M38);
B. a protein derived from the protein in A and formed by substituting, deleting or adding one or more (e.g., 1-20; preferably 1-15; more preferably 1-10, such as 5 or 3) amino acid residues in the amino acid sequence of the protein in A with the function of the protein in A, but with conserved positions corresponding to (a)-(t) defined in A (with the same amino acid);
C. a protein derived from the protein in A with more than 80% (preferably more than 85%; more preferably more than 90%; more preferably more than 95%, such as 98%, 99%) homology with the amino acid sequence of the protein in A and the function of the protein in A, but with conserved positions corresponding to (a)-(t) defined in A (with the same amino acid).

In one or more preferable embodiments, the stability comprises thermal stability.

In one or more preferable embodiments, the temperature stability or thermal stability comprises temperature stability or thermal stability at a temperature above 37°C, preferably above 40°C, more preferably above 42°C, more preferably above 45°C, more preferably above 48°C, and more preferably at or above 50°C.

In one or more preferable embodiments, the firefly luciferase mutant comprises a protein fragment without a signal peptide of SEQ ID NO: 2.

In another aspect, the present disclosure provides a polynucleotide, wherein the polynucleotide encodes the firefly luciferase mutant.

In another aspect, the present disclosure provides a vector or a genetically engineered host cell comprising the vector, wherein the vector comprises the polynucleotide; or the host cell comprises the vector or has the polynucleotide integrated into its genome.

In one or more preferable embodiments, the host cell comprises: prokaryotic cells or eukaryotic cells; preferably, the eukaryotic host cell comprises: yeast cells, fungal cells, insect cells, mammalian cells, etc.; the prokaryotic host cell comprises Escherichia coli, Bacillus subtilis, etc.

In one or more preferable embodiments, the host cell is a yeast cell.

In one or more preferable embodiments, the 5' end of the polynucleotide in the vector further comprises a signal peptide, a tag polypeptide, a fluorescent protein and/or a promoter. The 3' end of the polynucleotide further comprises a tag polypeptide, a fluorescent protein and/or a terminator.

In one or more preferable embodiments, compared to the firefly luciferase with the amino acid sequence shown in SEQ ID NO: 2, the enzyme activity of the modified firefly luciferase is increased by more than 20%; preferably by more than 50%; more preferably by more than 100%; more preferably by more than 150%.

In one or more preferable embodiments, compared to the firefly luciferase with the amino acid sequence shown in SEQ ID NO: 2, the thermal stability of the modified firefly luciferase is significantly improved by more than 1.6 times, preferably by more than 2.5 times, more preferably by more than 2.6 times, more preferably by more than 2.7 times, such as by 2.8 times.

In another aspect, the present disclosure provides a method for producing a firefly luciferase mutant, wherein the method comprises the following steps: (i) culturing the host cell; (ii) collecting a culture comprising the firefly luciferase mutant; (iii) isolating the firefly luciferase mutant from the culture.

In another aspect, the present disclosure provides a use of the firefly luciferase mutant, the host cell expressing the mutant or a lysate thereof, for catalyzing the oxidation of firefly luciferase substrate and generating ATP-dependent bioluminescence, or for detecting cell number or cell viability.

In another aspect, the present disclosure provides a method for catalytically oxidizing firefly luciferin to produce bioluminescence, comprising: catalytically oxidizing using the firefly luciferase mutant, a host cell expressing the mutant or a lysate thereof, and producing bioluminescence in the presence of ATP.

In one or more embodiments, the catalysis is carried out in a reaction-adapted system, comprising, for example, metal ions (such as divalent metal ions) and oxygen; specifically, the metal ions comprises, for example, magnesium ions, calcium ions, zinc ions and/or manganese ions.

In one or more embodiments, the reaction-adapted system further comprises reagents selected from the group consisting of reaction buffer, sodium chloride, detergent, gelatin, MES, quaternary ammonium salts such as CTAB, NaF, chelating agents such as EDTA, antifoaming agents such as DF204, thiol-containing compounds such as DTT, phosphates, sulfites and thiosulfates, BSA (excluding gelatin), glycerol and other protein stabilizers.

In one or more embodiments, the use or method is for in vitro ATP detection; preferably, the amount of ATP contained in a solution system is determined using the firefly luciferase mutant and luciferase substrate.

In one or more embodiments, the use or method is for cell viability detection; preferably, the firefly luciferase mutant and luciferase substrate are mixed with cell cultures to measure cell viability.

In one or more embodiments, the use or method is for cell number detection; preferably, the firefly luciferase mutant and luciferase substrate are mixed with cell cultures to measure the number of cells.

In another aspect, the present disclosure provides a detection system or detection kit for catalyzing the oxidation of firefly luciferin to produce bioluminescence, comprising: the firefly luciferase mutant, or the host cell, a culture thereof or a lysate thereof.

In one or more embodiments, the detection system or detection kit further comprises firefly luciferin.

In one or more embodiments, the detection system further comprises metal ions (such as divalent metal ions); preferably, the metal ions include magnesium ions, calcium ions, zinc ions and/or manganese ions.

In one or more embodiments, the detection system further comprises reagents selected from the group consisting of reaction buffer, sodium chloride, detergent, gelatin, MES, quaternary ammonium salt, chelating agent, defoaming agent, thiol-containing compound, phosphate, sulfite, thiosulfate, BSA (excluding gelatin), glycerol, and other protein stabilizers.

In one or more embodiments, the detection system further comprises a cell lysis reagent.

In one or more embodiments, the detection system further comprises an ATP extraction reagent.

In another aspect, the present disclosure provides an ATP tester, comprising: a container comprising the firefly luciferase mutant; a container comprising a luciferase substrate within it; sampling, dispensing and reaction devices used for adding a sample to be tested, wherein the sample to be tested is a sample to be tested by ATP detection; preferably, it further comprises a gas supply device, wherein the gas comprises oxygen.

Other aspects of the present disclosure will be apparent to those skilled in the art from the disclosure herein.

### DESCRIPTION OF DRAWINGS

Figure 1. Comparison of the predicted three-dimensional structure and the overall structure of firefly *(Photuris pensylvanica)* luciferase.
Figure 2. Analysis of the three-dimensional structure of firefly *(Photuris pensylvanica)* luciferase and illustration of the substrate-binding pocket.
Figure 3. Comparative analysis of enzyme activities among crude enzymes of the wild-type, 146-1H2 and mutants.
Figure 4. Electropherograms of the expressed and purified firefly luciferase mutants (including WT and 146-1H2) whose crude enzymes showed significant enzyme activity.
Figure 5. Comparison of enzyme activity assays for purified firefly luciferase.
Figure 6. Comparison of signal thermal stability of purified firefly luciferase.
Figure 7. Comparison of purified 146-1H2, M1 and M2 for ATP detection at various concentrations.
Figure 8. Comparison of signal thermal stability of purified 146-1H2, M1 and M2 when used for ATP detection at various concentrations.
Figure 9. Comparison of enzyme activity detection using purified M1 and M2 with different amounts of Hela cells.

### DETAILED DESCRIPTION

Firefly luciferase is a protein consisting of 545 amino acids, and the sites closely related to its enzymatic activity or thermal stability have not yet been fully elucidated. In previous work involving spatial structure analysis and mutagenesis, a large number of amino acid positions were considered, and a series of mutants related to the substrate-binding pocket of firefly luciferase were designed, constructed, and screened. After preliminary screening, 23 mutants with relatively high enzymatic activity were selected for protein expression and purification, followed by enzyme activity assays and thermal stability detections. Compared with the control (146-1H2), a series of firefly luciferase mutants showed varying degrees of improvement in enzymatic activity while maintaining good thermal stability. Among them, two mutants, M1 (F293L) and M2 (Y254S), exhibited significant increases in enzymatic activity, 182% and 172%, higher than that of the control (146-1H2), respectively. Moreover, incubation at 50 °C for 24 hours did not affect the enzymatic activity of these two mutant proteins, indicating their high thermal stability.

### Terms

As used herein, and unless otherwise specified, the terms "firefly luciferase mutant", "mutant firefly luciferase", "luciferase mutant" and "mutant luciferase" are used interchangeably and refer to enzymes (polypeptides/proteins) formed by mutating certain positions determined by the inventor to be correlated with enzyme activity/thermal stability of firefly luciferase, corresponding to a firefly luciferase before mutation, preferably, corresponding to the amino acid sequence shown in SEQ ID NO: 2. The mutation(s) are selected from the group consisting of position 293, position 254, position 229, or position 231, or combinations thereof, encompassing a total of 23 firefly luciferase mutants.

If it is necessary to represent the firefly luciferase (146-1H2) before mutation, it can be an enzyme with an amino acid sequence shown in SEQ ID NO: 2. Unless otherwise specified, the positions of the mutants in this disclosure are based on the sequence shown in SEQ ID NO: 2.

In the present disclosure, unless otherwise specified, the identification of firefly luciferase mutants employs the notation "amino acid substituted at original amino acid position" to denote the mutated amino acid in the firefly luciferase mutant. For instance, F293L indicates that the amino acid at position 293 has been replaced from F in the starting enzyme to L.

As used in this document, "isolated firefly luciferase" refers to a firefly luciferase mutant that essentially does not contain other proteins, lipids, carbohydrates, or other substances naturally associated with it. Those skilled in the art can purify the firefly luciferase mutant using standard protein purification methods. A substantially pure protein will produce a single major band on a non-reducing polyacrylamide gel.

As used herein, "improving enzyme activity or signal thermal stability" refers to a statistically significant improvement, or a significant increase, in the enzyme activity or signal thermal stability of a firefly luciferase mutant compared to the starting polypeptide of the firefly luciferase before mutation. For example, under the same reaction conditions/environment, after a certain period of reaction, the enzyme activity or signal thermal stability of the firefly luciferase mutant with improved enzyme activity or signal thermal stability is significantly higher than that of the enzyme before mutation by more than 5%, 10%, 20%, 30%, 50%, 70%, 80%, 100%, 150%, etc.

As used in this document, "library of firefly luciferase mutants or polynucleotides encoding them" refers to a collection of polypeptides comprising a series of firefly luciferase mutants or polynucleotides thereof provided by the present disclosure. Multiple firefly luciferase mutants with different enzymatic activities or varying signal thermal stabilities, or polynucleotides encoding them, are assembled in a library, facilitating a person skilled in the art to select a suitable firefly luciferase or its encoding nucleic acid according to the required reaction conditions.

As used herein, the terms "comprise" or "include" encompass "comprising", "consisting essentially of", and "consisting of". The term "consisting essentially of" means that, in addition to the essential components or ingredients, the composition/reaction system/kit may contain minor amounts of secondary components and/or impurities that do not materially affect the essential characteristics of the active ingredient(s).

As used herein, the term "effective amount" refers to an amount sufficient to elicit a function or enzymatic activity in a reaction of interest in the present disclosure and to achieve the intended effect (e.g., accurate detection results).

### Firefly luciferase mutants, encoding nucleic acids and constructs thereof

The inventor utilized a protein spatial structure prediction tool to predict the three-dimensional structure of the firefly (Photuris pensylvanica) luciferase, and conducted a comparative analysis with the published structure of the firefly luciferase-substrate complex in the Protein Data Bank (PDB). This comparison confirmed the uniqueness of the firefly luciferase mutant of the present disclosure.

The firefly luciferase mutant of the present disclosure can be a chemically synthesized product, or produced using recombinant technology from prokaryotic or eukaryotic hosts (e.g., bacterial, yeast, higher plant, insect, and mammalian cells).

The present disclosure also comprises fragments, derivatives and analogues of the firefly luciferase mutants. As used herein, the terms "fragment", "derivative", and "analogue" refer to proteins that maintain essentially the same biological function or enzymatic activity as the native firefly luciferase mutant of the present disclosure. The protein fragments, derivatives or analogues of the present disclosure can be (i) proteins with one or more conserved or non-conserved amino acid residues (preferably conserved amino acid residues) substituted, whereas such substituted amino acid residues may or may not be encoded by the genetic code, or (ii) proteins with substituent groups at one or more amino acid residues, or (iii) proteins formed by the fusion of additional amino acid sequences to the protein sequence (such as leader sequences or secretory sequences or sequences or proprotein sequences used for purifying the proteins or fused proteins). According to the definition herein, these fragments, derivatives and analogues fall within the scope known to those skilled in the art. However, the condition that must be met is that the amino acid sequence of the firefly luciferase mutant and the fragments, derivatives and analogues must comprise at least one mutation specifically indicated above in the present disclosure.

In the present disclosure, the term "firefly luciferase mutant" also comprises (but is not limited to): deletions, insertions and/or substitutions of several (typically 1-20, more preferably 1-10, and even more preferably 1-8, 1-5, 1-3, or 1-2) amino acids, as well as the addition or deletion of one or several (typically up to 20, more preferably up to 10, and even more preferably up to 5) amino acids at the C-terminus and/or N-terminus. For instance, in the art, substitutions with amino acids having similar or analogous properties typically do not alter the function of the protein. Similarly, the addition or deletion of one or several amino acids at the C-terminus and/or N-terminus usually does not alter the function of the protein. This term also comprises enzymatically active fragments and enzymatically active derivatives of the firefly luciferase mutant. However, among these variants, there must be at least one mutation as described above in the present disclosure.

In the present disclosure, the term "firefly luciferase mutant" also comprises (but is not limited to): derived proteins that retain their protease activity and share more than 80%, preferably more than 85%, more preferably more than 90%, further more preferably more than 95%, such as more than 98% or more than 99%, sequence identity with the amino acid sequence of the firefly luciferase mutant described. Similarly, among these derived proteins, there must be at least one mutation as described above in the present disclosure.

The disclosure also provides analogues of the firefly luciferase mutant. These analogues may differ from the firefly luciferase mutant in terms of amino acid sequence, or in modified forms that do not affect the sequence, or both. These polypeptides comprise natural or induced genetic variants. Induced variants can be obtained through various methods, such as random mutagenesis by radiation or exposure to mutagens, as well as site-directed mutagenesis or other known molecular biological methods. Analogues also comprise those with residues different from natural L-amino acids (such as D-amino acids), and those with non-naturally occurring or synthetic amino acids (such as β, γ-amino acids). It should be understood that the polypeptides of the present disclosure are not limited to the representative polypeptides exemplified above. Forms of modification (usually without altering the primary structure) comprise chemical derivatization of polypeptides in vivo or in vitro, such as acetylation or carboxylation. Modifications also comprise glycosylation, such as those that occur during the synthesis and processing of polypeptides or further processing steps. Such modifications can be achieved by exposing polypeptides to enzymes that catalyze glycosylation, such as mammalian glycosylases or deglycosylases. Forms of modification also comprise sequences with phosphorylated amino acid residues (such as phosphotyrosine, phosphoserine, phosphothreonine). Polypeptides that are modified to enhance their resistance to proteolysis or optimize their solubility properties are also included.

The present disclosure also provides polynucleotide sequences encoding the firefly luciferase mutant or conservative variant proteins of the disclosure.

The polynucleotides of the present disclosure can exist in the form of DNA or RNA. Forms of DNA comprise cDNA, genomic DNA, or artificially synthesized DNA. DNA can be single-stranded or double-stranded. DNA can be a coding strand or a non-coding strand.

The polynucleotides encoding the mature protein of the mutants comprise: coding sequences that only encode the mature protein; coding sequences for the mature protein and various additional coding sequences; coding sequences for the mature protein (and optional additional coding sequences) as well as non-coding sequences.

"Polynucleotides encoding a protein" can refer to polynucleotides solely encoding this protein, or it can also refer to polynucleotides that comprise additional coding and/or non-coding sequences.

The present disclosure also relates to vectors comprising the polynucleotides of the present disclosure, as well as host cells produced through genetic engineering using the vectors of the present disclosure or the coding sequence of the firefly luciferase mutant, and methods for producing the mutant enzymes described in the present disclosure through recombinant technology.

Through conventional recombinant DNA technology, the polynucleotide sequences of the present disclosure can be expressed or produced. Generally speaking, steps are as follows:
(1). transforming or transducing an appropriate host cell with a polynucleotide (or variants) encoding the firefly luciferase mutant of the present disclosure, or with a recombinant expression vector containing the polynucleotide;
(2). culturing the host cells in a suitable medium;
(3). isolating and purifying proteins from the culture medium or cells.

In the present disclosure, the polynucleotide sequence of the firefly luciferase mutant can be inserted into a recombinant expression vector. The term "recombinant expression vector" refers to bacterial plasmids, phages, yeast plasmids, plant cell viruses, mammalian cell viruses or other vectors well known in the art. Any plasmid and vector can be used as long as it can replicate and be stable in the hosts. An important characteristic of an expression vector is that it usually contains an origin of replication, a promoter, a marker gene, and translation control elements.

Those skilled in the art are well known of methods for constructing expression vectors containing the DNA sequence encoding the firefly luciferase mutant and suitable transcription/translation control signals. These include in vitro recombinant DNA techniques, DNA synthesis techniques, in vivo recombination techniques, etc. The DNA sequence can be effectively ligated to an appropriate promoter in an expression vector to direct mRNA synthesis. The expression vector also includes a ribosome binding site for translation initiation and a transcription terminator. Preferably, the expression vector comprises one or more selectable marker genes to provide phenotypic traits for selecting transformed host cells.

Vectors comprising the appropriate DNA sequences described above, together with appropriate promoter or control sequences, can be used to transform appropriate host cells so that they can express the protein.

In the present disclosure, the host cell can be a prokaryotic cell, such as a bacterial cell; a lower eukaryotic cell, such as a fungal cell or a yeast cell; or a higher eukaryotic cell, such as a plant cell. Representative examples are: Escherichia coli, Bacillus subtilis, Streptomyces, and Agrobacterium; as well as eukaryotic cells such as yeast and plant cells. In specific examples of the present disclosure, Escherichia coli is used as the host cell.

Those skilled in the art are well aware of how to select appropriate vectors, promoters, enhancers, and host cells. In the preferred embodiment of the present disclosure, the expression vector used is pET22b; the microbial host cells transformed by the expression vector are all Escherichia coli.

The obtained transformants can be cultured using conventional methods to express the polypeptides encoded by the genes of the present disclosure. Depending on the host cell used, the culture medium can be selected from various conventional media. Culturing is carried out under conditions suitable for the growth of the host cell. Once the host cell reaches an appropriate cell density, the selected promoter is induced using an appropriate method (such as temperature shift or chemical induction), and the cells are cultured for a further period of time.

The recombinant polypeptides in the above methods can be expressed in cells, on the cell membrane or secreted outside the cells. If necessary, the recombinant proteins can be separated and purified using various separation methods based on their physical, chemical and other characteristics. These methods are well known to those skilled in the art. Examples of these methods comprise, but are not limited to: conventional renaturation treatment, treatment with protein precipitants (salting-out method), centrifugation, osmotic lysis, ultrasonication, ultracentrifugation, molecular sieve chromatography (gel filtration), adsorption chromatography, ion exchange chromatography, high-performance liquid chromatography (HPLC) and various other liquid chromatography techniques, as well as combinations of these methods.

### Uses of firefly luciferase mutants

Under suitable reaction conditions, firefly luciferase can catalyze the oxidation of firefly luciferin, resulting in bioluminescence. That is, the firefly luciferase of the present disclosure can be applied to the ATP bioluminescence assay as a catalyst. The ATP bioluminescence assay is a simplified biochemical method that utilizes the reaction between ATP and the luciferin-luciferase complex to determine the presence of adenosine triphosphate (ATP). This method can be used for the determination of ATP levels in vitro or in cells. The bioluminescence reaction requires ATP, luciferin and firefly luciferase. During the reaction, luciferin is oxidized and emits fluorescence, and the number of photons can be measured using an ATP fluorometer, which is directly proportional to the ATP content. When applied to cell viability assays, since the ATP content in each cell is constant, the ATP content in the sample is related to the number of cells in the sample. This method allows for the determination of ATP levels in a very short time (e.g., seconds). Using the firefly luciferase mutant of the present disclosure, detection can be achieved more efficiently and sensitively.

After obtaining the firefly luciferase mutant enzyme of the present disclosure, according to the guidance of the disclosure, those skilled in the art can conveniently utilize the enzyme to catalyze the oxidation of firefly luciferin and produce bioluminescence in the presence of ATP. In a preferred embodiment, the catalysis is carried out in a suitable reaction system, including metal ions (such as divalent metal ions, specifically magnesium ions) and oxygen; preferably, the suitable reaction system also includes (but is not limited to) reagents selected from the group consisting of reaction buffer, sodium chloride, detergent, gelatin, MES, quaternary ammonium salt (such as CTAB), NaF, chelating agent (such as EDTA), defoaming agent (such as DF204), thiol-containing compound (such as DTT), phosphate, sulfite, thiosulfate, protein stabilizers other than gelatin such as BSA, glycerol, etc.

The ATP bioluminescence method based on firefly luciferase has a wide range of applications, spanning various fields such as food (e.g., microbial detection), pharmaceuticals, electronics, and cosmetics. For instance, the bioluminescence method is employed to determine bacterial contamination in meat products. Additionally, the ATP bioluminescence method can be used for the detection of lactic acid bacteria in dairy products, the determination of total bacterial count in beer, and bacteriological testing of condiments and dehydrated vegetables. Furthermore, the ATP bioluminescence method is applicable for cleanliness inspection in food production environments, such as assessing the cleanliness of food production lines, kitchens, refrigerators, food preparation stations, and railway station food utensils. Additionally, the ATP bioluminescence method can be used for cleanliness inspection in medical settings, such as microbial testing of surgical instruments and medical consumables.

For animal and plant tissue samples or cell samples, the method of the present disclosure can also be applied to detect ATP content, in order to study biological metabolic performance. For example, in the field of botany involving transgenic research, the ATP content of plant callus tissues can be measured to study the characteristics of cellular energy metabolism therein.

It should be understood that the firefly luciferase mutant of the present disclosure with higher enzyme activity and thermal stability, is suitable for a wide range of ATP detection methods known or being developed in the field, exhibiting broad applicability.

In a specific example of the present disclosure, numerous Pennsylvania firefly luciferase mutants were established based on Luc146-1H2, and corresponding prokaryotic expression plasmids were constructed. After sequencing confirmation, the mutant plasmids were transformed into competent Escherichia coli cells simultaneously with wild-type (WT) and control (146-1H2) plasmids. After cultivation and induction, E. coli broths expressing firefly luciferase were obtained; the cells were lysed to release firefly luciferase, and the enzyme activities of different mutants were compared. Twenty-three mutants with higher enzyme activity were cultured and induced for expression, and firefly luciferase proteins were purified. The Luc146-1H2 enzyme activity and signal thermal stability of the mutants and the control were compared.

This disclosure employs a method combining molecular theoretical design and functional screening to obtain multiple firefly luciferase mutants with higher enzyme activity, all of which can be effectively used for quantitative detection of ATP. Among them, the enzyme activity of mutant M1 (F293L) is approximately 182% higher than that of the control firefly luciferase, and the enzyme activity of mutant M2 (Y254S) is approximately 172% higher than that of the control firefly luciferase. When the purified mutants and controls were heated at 50°C for 24 hours, and the enzyme activity before and after heating was compared, it was found that treatment at 50°C for 24 hours did not significantly affect the enzyme activity of firefly luciferase. Further testing revealed that this series of luciferase mutants can also be used for the detection of cell number and enzyme activity.

### Composition/kit of firefly luciferase mutants

The present disclosure further provides a composition comprising an effective amount of the firefly luciferase mutant of the disclosure, as well as other components required for detection. Such components comprise, but are not limited to, those selected from the group consisting of firefly luciferin (as a substrate), metal ions (such as divalent metal ions), reaction buffer, sodium chloride, detergents, gelatin, MES, quaternary ammonium salts (such as CTAB), NaF, chelating agents (such as EDTA), antifoams (such as DF204), thiol-containing compounds (such as DTT), phosphates, sulfites, thiosulfates, protein stabilizers other than gelatin such as BSA, glycerol, etc. Those skilled in the art can determine the effective amount of the firefly luciferase mutant in the composition based on its actual effective amount. Other substances that further modulate the enzyme activity of the firefly luciferase mutant of the disclosure or promote the reaction process can also be added to the composition.

The present disclosure further provides a detection system, comprising: the firefly luciferase mutant of the present disclosure, and firefly luciferin (as a substrate); preferably, it also comprises components selected from the group consisting of metal ions (such as divalent metal ions), reaction buffer, sodium chloride, detergent, gelatin, MES, quaternary ammonium salt (such as CTAB), NaF, chelating agent (such as EDTA), defoaming agent (such as DF204), thiol-containing compound (such as DTT), phosphate, sulfite, thiosulfate, protein stabilizers other than gelatin such as BSA, glycerol, etc. In this detection system, when the sample to be tested is added, a reaction can occur in a very short period of time, and the measurement result can be obtained.

To facilitate extensive or commercial applications, the present disclosure further provides a detection system or detection kit, comprising: the firefly luciferase mutant of the present disclosure, firefly luciferin (as a substrate); preferably, it also comprises components selected from the group consisting of metal ions (such as divalent metal ions), reaction buffer, sodium chloride, detergent, gelatin, MES, quaternary ammonium salt (such as CTAB), NaF, chelating agent (such as EDTA), defoaming agent (such as DF204), thiol-containing compound (such as DTT), phosphate, sulfite, thiosulfate, BSA other than gelatin, glycerol, and other protein stabilizers. Among them, the firefly luciferase mutant, luciferin, metal ions (such as divalent metal ions), reaction buffer, sodium chloride, detergent, gelatin, MES, and other reagents are independently placed in different containers, or two or more of them are mixed in the same container.

As a preferable embodiment of the present disclosure, the detection kit may further comprise: a cell lysis reagent, and/or an ATP extraction reagent.

As a preferable embodiment of the present disclosure, the detection kit may further comprise an instruction manual to guide people in applying the kit of the present disclosure in the correct manner.

To facilitate widespread or commercial application, the present disclosure further provides an ATP tester, comprising: a container comprising the firefly luciferase mutant; a container comprising a luciferase substrate (such as luciferin) within it; sampling (e.g., swab), dispensing and reaction devices used for adding a sample to be tested, wherein the sample to be tested is a sample to be tested by ATP detection; and a gas supply device, wherein the gas comprises oxygen. Alternatively, air (oxygen) present in its natural state can be used to replace the gas supply device.

As a preferred embodiment of the present disclosure, the ATP tester further comprises: a container, and contained within the container are: metal ions (such as divalent metal ions) (solution), reaction buffer, sodium chloride, detergent, gelatin, MES, quaternary ammonium salt (such as CTAB), NaF, chelating agent (such as EDTA), defoaming agent (such as DF204), thiol-containing compound (such as DTT), phosphate, sulfite, thiosulfate, protein stabilizers other than gelatin such as BSA, glycerol, etc. In the ATP tester, the reagents such as the firefly luciferase mutant, luciferin, metal ions (such as divalent metal ions), reaction buffer, sodium chloride, detergent, gelatin, MES, etc., are independently placed in different containers, or two or more of them are mixed in the same container. The ATP tester may be equipped with some calculation programs and an output screen, thereby visually displaying the measurement results to people.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### I. Experimental materials, reagents and instruments

Escherichia coli DH5α, BL21(DE3), gene site-directed mutagenesis kit, endonucleases Dpn I, Nde I, Xho I, LB medium-related reagents, antibiotics, inducers, plasmid mini-extraction kit, Ni affinity chromatography column packing, ATP standard, firefly luciferase detection reagent, 96-well plate, and other materials and reagents are all commercialized products/reagents from Shanghai Beyotime Biotechnology Co., Ltd.

The gene random mutation kit was purchased from Takara.

The PCR machine was purchased from Bio-Rad.

The ultrasonic cell disruptor was purchased from Ningbo XinZhi Biotechnology Co., Ltd.

The high-pressure cell disruptor was purchased from Antares Nanotech (Suzhou) Co., Ltd.

The Clinx gel imager was purchased from Shanghai Qinxiang Scientific Instrument Co., Ltd.

The multi-function microplate reader Varioskan LUX was purchased from ThermoFisher.

### II. Experimental Methods

### 1. Structure prediction and analysis of luciferase from the wild-type Pennsylvania firefly (Photuris pensylvanica) and the Luc146-1H2 mutant

The three-dimensional structure of a protein can visually reveal information such as the regions of protein-substrate interaction, the modes of substrate binding and release and the key amino acid positions involved in substrate-specific recognition. This information is of great guiding significance for the targeted design of mutation(s) to modify the firefly (*Photuris pensylvanica*) luciferase. Currently, there is no resolved spatial structure data for the firefly (*Photuris pensylvanica*) luciferase. With the help of protein three-dimensional structure prediction tools, its overall structure is predicted and key enzyme active site analysis and experimental analysis are conducted. Based on the analysis results, it is speculated that amino acid positions involved in substrate recognition and binding, as well as those affecting the thermal stability of luciferase, may be involved. On this basis, a series of site-directed mutants are constructed based on the Luc146-1H2 mutant, and their enzyme activity and thermal stability are tested, with the expectation of selecting mutants that can significantly improve enzyme activity and thermal stability. The protein sequences of wild-type and Luc146-1H2 mutant firefly luciferases are compared in the "Sequence Information" part.

### 2. Construction of site-directed mutants of luciferase

The 146-1H2 gene (with a seuquence as shown in "Sequence Information") was obtained through gene synthesis and cloned into the pET22b expression vector via the NdeI and XhoI restriction sites. A 6X His purification tag and a stop codon were added to the C-terminus of the protein, resulting in the pET22b-luc146-1H2 plasmid.

Using the pET22b-luc146-1H2 plasmid as a template, PCR primers for gene site-specific mutation were designed and synthesized. The gene site-specific mutation PCR was performed using the QuickMutation^{™} Gene Site-Specific Mutation Kit (Beyotime, D0206).

The 50µl reaction system comprises: 29µl of nuclease-free water, 5µl of 10X BeyoFusion^{™} Buffer (containing Mg²⁺), 10µl of dNTP mix (2.5mM for each), 2µl of forward primer (10µM), 2µl of reverse primer (10µM), 1µl of pET22b-luc146-1H2 plasmid (200ng) and 1µl of BeyoFusion^{™} DNA polymerase.

The PCR procedure is as follows: 95°C pre-denaturation for 3 minutes; 20 cycles: 95°C for 30 seconds, 55°C for 30 seconds, 68°C for 3 minutes (30 seconds/kb); a continuous extension at 68°C for 15 minutes; and a temporary storation at 4°C.

After the PCR reaction was complete, 1 µl of endonuclease Dpn I (Beyotime, D6257) was added to the PCR reaction system, mixed well, and incubated at 37°C for 1 hour to digest the template plasmid.

Following digestion, 10 µL of each PCR product was added to 100 µL of DH5α supercompetent cells (Beyotime, D1031). The mixture was gently mixed and then placed on ice for 30 minutes, followed by a heat shock at 42°C for 90 seconds and an incubation on ice for 2 minutes.

900 µL of antibiotic-free LB medium was added and the cells were shaken at 37°C for 1 hour. The bacterial cells were collected by centrifugation, resuspended in 100 µL of antibiotic-free LB medium, and the suspension was then spread evenly onto LB plates containing 100 µg/mL ampicillin (Beyotime, ST007). The plates were inverted and incubated in a 37°C incubator overnight.

The following day, single colonies were picked and inoculated into 10 mL of LB liquid medium containing 100 µg/mL ampicillin, followed by overnight culture. Plasmids were extracted from the bacterial cultures of each mutant using a plasmid miniprep kit (Beyotime, D0003) and plasmid concentrations were determined.

The target genes were sequenced using the universal primers T7 and T7-Terminator. For mutants requiring the introduction of multiple mutation(s), multiple rounds of site-directed mutagenesis were performed. Each round introduced one or several mutation(s) and sequencing verification confirming success was required before proceeding to the next round.

### 3. Construction of random mutants of luciferase

To maximize the potential for improving the performance of the Luc146-1H2 mutant and to screen for a greater number of high-quality mutants, gene random mutagenesis PCR was performed using the Diversify^{®} PCR Random Mutagenesis Kit with the pET22b-luc146-1H2 plasmid as the template, concurrent with the site-directed mutagenesis efforts. This method aimed to construct random mutants and subsequently screen them for enzyme activity and thermal stability.

The 50 µL reaction mixture for the random mutagenesis PCR comprises: 35 µL PCR Grade Water, 5 µL 10× TITANIUM Taq Buffer, 4 µL MnSO₄ (8 mM), 1 µL dGTP (2 mM), 1 µL 50× Diversify dNTP Mix, 1 µL forward primer (10 µM, sequence: 5'-gtgctcgagttagtggtgatggtg-3' (SEQ ID NO: 6)), 1 µL reverse primer (10 µM, sequence: 5'-agatatacatatggcagataagaatattt-3' (SEQ ID NO: 7)), 1 µL pET22b-luc146-1H2 plasmid (1 ng) and 1 µL TITANIUM Taq DNA polymerase.

The PCR procedure is as follows: 94°C pre-denaturation for 30s; 25 cycles: 94°C for 30s, 45°C for 30s, 68°C for 2min; a continuous extension at 68°C for 1min; and a temporary storation at 4°C.

Fragment digestion: The 50µl reaction system comprises: PCR Grade Water, 5µl 10x Buffer R, 1µl NdeI (Beyotime, D6485), 1µl XhoI (Beyotime, D6721) and PCR product (2µg). Digestion is performed at 37°C overnight.

The overnight enzyme digestion product was ligated to the NdeI and XhoI enzyme-digested linearized pET22b vector and the ligation product was transformed into BL31(DE3) competent cells.

Monoclonal antibodies were picked from the plate for protein expression and the enzyme activity of crude luciferase from different mutants was detected using Bac-Lumi^{™} bacterial firefly luciferase detection reagent (Beyotime, RG039).

### 4. Enzyme activity detection of crude luciferase mutants

The Bac-Lumi^{™} Bacterial Firefly Luciferase Assay Reagent (Beyotime, RG039) was thawed in advance and equilibrated to room temperature. To prevent signal saturation beyond the linear detection range of the microplate reader, the induced bacterial culture was diluted 10-fold with LB medium. Then, 100 µL of the diluted culture was transferred to a BeyoGold^{™} solid white 96-well cell culture plate (flat bottom with lid, individually packaged) (Beyotime, FCP968), with 100 µL of LB medium used as a blank control. To each well, 100 µL of the Bacterial Firefly Luciferase Assay Reagent was added. The 96-well plate was placed on a horizontal decolorizing shaker and incubated with shaking at room temperature for 10 minutes. Chemiluminescence was then measured using a multifunctional microplate reader, with readings taken every 2 minutes over a total period of 2 hours. The relative light unit (RLU) signal intensity can roughly reflect the enzyme activity levels of the different mutants.

### 5. Expression of high-enzyme activity firefly luciferase mutants

Using the super competent bacterial preparation kit (Beyotime, D0302), Escherichia coli BL21(DE3) glycerol bacteria (Beyotime, D0337) were prepared into competent cells. The correctly sequenced mutant, wild-type, and control plasmids were transformed into the competent E. coli BL21(DE3) cells and cultured overnight at 37°C on LB plates containing ampicillin. The next day, single colonies were picked and transferred to 10 ml of LB liquid medium containing ampicillin. The cultures were incubated at 37°C, 220 rpm in a shaker until the OD600 value reached between 0.6 and 0.8. Then, IPTG (Beyotime, ST098) was added at a final concentration of 0.1 mM, and the cultures were incubated at 18°C, 220 rpm for 16 hours to induce protein expression. 10ml of bacterial cultures before and after induction were taken, and the cells were collected by centrifugation. The pellets were resuspended in 10 ml of PBS. After being lysed in an ultrasonic cell disruptor for 10 minutes, the lysates were centrifuged at 12,000 rpm for 10 minutes. 50µl of the supernatant after centrifugation were taken, and 10 µl of 6X SDS-PAGE protein loading buffer (Beyotime, P0015F) was added. The samples were heated in a boiling water bath at 100°C for 10 minutes and then centrifuged at 12,000 rpm for 10 minutes. 20µl of each sample were taken for SDS-PAGE electrophoresis to observe whether firefly luciferase protein was expressed in the supernatant.

### 6. Purification of high-enzyme activity firefly luciferase mutants

Based on the results of crude enzyme activity assay, 23 firefly luciferase mutants with significantly enhanced enzyme activity were selected and each was expressed in BL21(DE3) strain at 1L scale compared to the control (146-1H2). The following purification steps were all conducted at 4°C. After induction, the bacterial cells were collected and resuspended in 100ml of lysis buffer (50mM NaH₂PO₄ pH8.0, 300mM NaCl, 10% glycerol, 1mM PMSF), and then lysed three times using a high-pressure cell disruptor at 850bar. The supernatant was collected after centrifugation at 12000rpm for 30min, and the target protein was purified using a BeyoGold^{™} His-tag Purification Resin (reduced-resistant chelate type) (Beyotime, P2218) affinity column. The purified firefly luciferase was dialyzed against enzyme storage buffer (25mM Tris-acetate pH7.8, 1mM EDTA, 1mM DTT, 0.2M (NH₄)₂SO₄, 15% glycerol, 30% ethylene glycol). The protein concentration was determined using a Bradford protein concentration assay kit (detergent-compatible type) (Beyotime, P0006) and confirmed by SDS-PAGE. The aliquoted protein was flash frozen in liquid nitrogen and stored at -80°C.

### 7. Enzyme activity and ATP quantitative detection of purified firefly luciferase mutant

The luciferase component was removed from the commercially available CellTiter-Lumi^{™} Luminescent Cell Viability Assay Kit (Beyotime, C0065) to obtain a new detection reagent, referred to as CTL. Purified firefly luciferase mutants and the control were diluted to 1mg/mL using CTL. ATP standard (Beyotime, D7378) was diluted with PBS to concentrations of 2, 1.5, 1, 0.5, 0.25, and 0.125µM. A 200µL reaction system was prepared in a 96-well plate: 5µL of 1 mg/mL firefly luciferase protein, 95µL of CTL, and 100 µL of ATP standard at varying concentrations (0-2µM, added last). The plate was placed on a horizontal decolorizing shaker and incubated with shaking at room temperature for 10 minutes. Chemiluminescence was then measured using a multifunctional microplate reader, with readings taken every minute over a total period of 20 minutes.

### 8. Thermal stability assay of purified firefly luciferase mutant

Firefly luciferase mutants and the control were diluted to 1 mg/mL using enzyme storage buffer. For each sample, 100µL (two aliquots of 50µL) was placed in a PCR instrument and incubated at 50°C for 24 hours. After incubation, the samples were centrifuged at 12,000 rpm for 10 minutes, and the supernatant was collected. The enzyme activity of the heated samples was measured alongside unheated proteins to compare the activity of heated versus unheated firefly luciferase.

### 9. Use of purified firefly luciferase mutants for cell viability detection

Purified mutants M1 and M2 were used to detect ATP content in different numbers of HeLa cells as an indicator of cell viability. Cultured HeLa cells were serially diluted with culture medium to generate a concentration gradient and incubated with 1% Triton X-100 to achieve complete lysis. For chemiluminescence detection in a 96-well plate, 100 µL of each HeLa cell concentration was seeded per well, corresponding to 10, 20, 50, 100, 200, 500, 1000, 2000, 5000, 10000, 20000, and 50000 cells per reaction. Wells containing culture medium without cells were included as negative controls. Cell viability was assessed by performing luminescence reactions in a 200µL system containing a final concentration of 25µg/mL of the luciferase mutant.

### 10. Sequence information

**WT amino acid sequence (SEQ ID NO: 1):**
**146-1H2 amino acid sequence (SEQ ID NO: 2):** 
MADKNILYGPEPFYPLEDGTAGEQMFDALSRYAAIPGCIALTNAHTKENVLYEEFLKLSCRLAESFKKYGL
KQNDTIAVCSENSLQFFLPVIASLYLGIIVAPVNDKYIERELIHSLGIVKPRIVFCSKNTFQKVLNVKSKLKSIETIII
LDLNEDLGGYQCLNNFISQNSDSNLDVKKFKPYSFNRDDQVASIMFSSGTTGLPKGVMLTHKNIVARFSIAKDPT
FGNAINPTSAILTVIPFHHGFGMMTTLGYFTCGFRVVLMHTFEEKLFLQSLQDYKVESTLLVPTLMAFLAKSALV
 
EKYDLSHLKEIASGGAPLSKEIGEMVKKRFKLNFVRQGYGLTETTSAVLITPKGDAKPGSTGKIVPLHAVKVVDP
TTGKILGPNEPGELYFKGPMIMKGYYNNEEATKAIIDNDGWLRSGDIAYYDNDGHFYIVDRLKSLIKYKGYQVA
PAEIEGILLQHPYIVDAGVTGIPDEAAGELPAAGVVVQTGKYLNEQIVQDYVASQVSTAKWLRGGVKFLDEIPK
GSTGKIDRKVLRQMLEKHTNG
**146-1H2 nucleotide sequence (SEQ ID NO: 3):** 
atggcagataagaatattttatatgggcccgaaccattttatcccttggaagatgggacggctggagaacagatgtttgacgcattatctcgttatgcagctattccgg
gctgcatagcattgacaaatgctcatacaaaagaaaatgttttatatgaagagtttctgaaactgtcgtgtcgtttagcggaaagttttaaaaagtatggattaaaacaaaacg
acacaatagcggtgtgtagcgaaaatagtctgcaatttttccttcctgtaattgcatcattgtatcttggaataattgtggcacctgttaacgataaatacattgaacgtgaatta
atacacagtcttggtattgtaaaaccacgcatagttttttgctccaagaatacttttcaaaaagtactgaatgtaaaatctaaattaaaatctattgaaactattattatattagactt
aaatgaagacttaggaggttatcaatgcctcaacaactttatttctcaaaattccgatagtaatctggacgtaaaaaaatttaaaccctattcttttaatcgagacgatcaggttg
cgtcgattatgttttcttctggtacaactggtctgccgaagggagtcatgctaactcacaagaatattgttgcacgattttctattgcaaaagatcctacttttggtaacgcaatt
aatcccacgtcagcaattttaacggtaatacctttccaccatggttttggtatgatgaccacattaggatactttacttgtggattccgagttgttctaatgcacacgtttgaaga
aaaactatttctacaatcattacaagattataaagtggaaagtactttacttgtaccaacattaatggcatttcttgcaaaaagtgcattagttgaaaagtacgatttatcgcactt
aaaagaaattgcatctggtggcgcacctttatcaaaagaaattggggagatggtgaaaaaacggtttaaattaaactttgtcaggcaagggtatggattaacagaaaccact
tcggctgttttaattacaccgaaaggtgacgccaaaccgggatcaactggtaaaatagtaccattacacgctgttaaagttgtcgatcctacaacaggaaaaattttggggc
caaatgaacctggagaattgtattttaaaggcccgatgataatgaagggttattataataatgaagaagctactaaagcaattattgataatgacggatggttgcgctctggtg
atattgcttattatgacaatgatggccatttttatattgtggacaggctgaagtcactgattaaatataaaggttatcaggttgcacctgctgaaattgagggaatactcttacaa
catccgtatattgttgatgccggcgttactggtataccggatgaagccgcgggcgagcttccagctgcaggtgttgtagtacagactggaaaatatctaaacgaacaaatc
gtacaagattatgttgccagtcaagtttcaacagccaaatggctacgtggtggggtgaaatttttggatgaaattcccaaaggatcaactggaaaaattgacagaaaagtgtt
aagacaaatgttagaaaaacacaccaatggg
**M1 (F293L) amino acid sequence (SEQ ID NO: 4):** 
MADKNILYGPEPFYPLEDGTAGEQMFDALSRYAAIPGCIALTNAHTKENVLYEEFLKLSCRLAESFKKYGL
KQNDTIAVCSENSLQFFLPVIASLYLGIIVAPVNDKYIERELIHSLGIVKPRIVFCSKNTFQKVLNVKSKLKSIETIII
LDLNEDLGGYQCLNNFISQNSDSNLDVKKFKPYSFNRDDQVASIMFSSGTTGLPKGVMLTHKNIVARFSIAKDPT
FGNAINPTSAILTVIPFHHGFGMMTTLGYFTCGFRVVLMHTFEEKLFLQSLQDYKVESTLLVPTLMALLAKSALV
EKYDLSHLKEIASGGAPLSKEIGEMVKKRFKLNFVRQGYGLTETTSAVLITPKGDAKPGSTGKIVPLHAVKVVDP
TTGKILGPNEPGELYFKGPMIMKGYYNNEEATKAIIDNDGWLRSGDIAYYDNDGHFYIVDRLKSLIKYKGYQVA
PAEIEGILLQHPYIVDAGVTGIPDEAAGELPAAGVVVQTGKYLNEQIVQDYVASQVSTAKWLRGGVKFLDEIPK
GSTGKIDRKVLRQMLEKHTNG
**M2 (Y254S) amino acid sequence (SEQ ID NO: 5):** 
MADKNILYGPEPFYPLEDGTAGEQMFDALSRYAAIPGCIALTNAHTKENVLYEEFLKLSCRLAESFKKYGL
KQNDTIAVCSENSLQFFLPVIASLYLGIIVAPVNDKYIERELIHSLGIVKPRIVFCSKNTFQKVLNVKSKLKSIETIII
LDLNEDLGGYQCLNNFISQNSDSNLDVKKFKPYSFNRDDQVASIMFSSGTTGLPKGVMLTHKNIVARFSIAKDPT
FGNAINPTSAILTVIPFHHGFGMMTTLGSFTCGFRVVLMHTFEEKLFLQSLQDYKVESTLLVPTLMAFLAKSALV
EKYDLSHLKEIASGGAPLSKEIGEMVKKRFKLNFVRQGYGLTETTSAVLITPKGDAKPGSTGKIVPLHAVKVVDP
TTGKILGPNEPGELYFKGPMIMKGYYNNEEATKAIIDNDGWLRSGDIAYYDNDGHFYIVDRLKSLIKYKGYQVA
PAEIEGILLQHPYIVDAGVTGIPDEAAGELPAAGVVVQTGKYLNEQIVQDYVASQVSTAKWLRGGVKFLDEIPK
GSTGKIDRKVLRQMLEKHTNG
**M3 (Q73H) amino acid sequence:**
   Based on SEQ ID NO: 2, the amino acid Q at position 73 is mutated to H.
**M4 (G158E, N402S, Y462N) amino acid sequence:**
   Based on SEQ ID NO: 2, the amino acid G at position 158 is mutated to E, the amino acid N at position 402 is mutated to S and the amino acid Y at position 462 is mutated to N.
**M5 (V239A) amino acid sequence:**
   Based on SEQ ID NO: 2, the amino acid V at position 239 is mutated to A.
**M6 (K379R) amino acid sequence:**
   Based on SEQ ID NO: 2, the amino acid K at position 379 is mutated to R.
**M7 (I145V, M248K, T351S, K532I) amino acid sequence:**
   Based on SEQ ID NO: 2, the amino acid I at position 145 is mutated to V, the amino acid M at position 248 is mutated to K, the amino acid T at position 351 is mutated to S and the amino acid K at position 532 is mutated to I.
   Among them, the amino acid at position 145 is V in WT and I145V corresponds to a mutation back to the residue at the corresponding position in WT.
**M8 (N130S, K212E) amino acid sequence:**
   Based on SEQ ID NO: 2, the amino acid N at position 130 is mutated to to S and the amino acid K at position 212 is mutated to E.
**M9 (F293L, V365I, G378V) amino acid sequence:**
   Based on SEQ ID NO: 2, the amino acid F at position 293 is mutated to L, the amino acid V at position 365 is mutated to I and the amino acid G at position 378 is mutated to V.
**M10 (M208T, I311F, E321G, L538F) amino acid sequence:**
   Based on SEQ ID NO: 2, the amino acid M at position 208 is mutated to T, the amino acid I at position 311 is mutated to F, the amino acid E at position 321 is mutated to G and the amino acid L at position 538 is mutated to F.
**M11 (N74K) amino acid sequence:**
   Based on SEQ ID NO: 2, the amino acid N at position 74 is mutated to K.
**M12 (Q277L, K379R, N413S, T488S, N543Y) amino acid sequence:**
   Based on SEQ ID NO: 2, the amino acid Q at position 277 is mutated to L, the amino acid K at position 379 is mutated to R, the amino acid N at position 413 is mutated to S, the amino acid T at position 488 is mutated to S and the amino acid N at position 543 is mutated to Y.
   Among them, the amino acid at position 413 of WT is K, while the position 413 of 146-1H2 is K413N, where N413 is mutated to S.
   The amino acid at position 543 of WT is S, while the position 543 of 146-1H2 is S543N, where the N543 is mutated to Y.
**M13 (N137K, A235T, K280R, N333D, I422V) amino acid sequence:**
   Based on SEQ ID NO: 2, the amino acid N at position 137 is mutated to K, the amino acid A at position 235 is mutated to T, the amino acid K at position 280 is mutated to R, the amino acid N at position 333 is mutated to D and the amino acid I at position 422 is mutated to V.
**M14 (K57R) amino acid sequence:**
   Based on SEQ ID NO: 2, the amino acid K at position 57 is mutated to R.
**M15 (T42A, E282D, K392I, D426V) amino acid sequence:**

   Based on SEQ ID NO: 2, the amino acid T at position 42 is mutated to A, the amino acid E at position 282 is mutated to D, the amino acid K at position 392 is mutated to I and the amino acid D at position 426 is mutated to V.
**M16 (E110G, V281E, I364V, E404G) amino acid sequence:**

   Based on SEQ ID NO: 2, the amino acid E at position 110 is mutated to G, the amino acid V at position 281 is mutated to E, the amino acid I at position 364 is mutated to V and the amino acid E at position 404 is mutated to G.
**M17 (S525P) amino acid sequence:**
   Based on SEQ ID NO: 2, the amino acid S at position 525 is mutated to P.
**M18 (E64V, N186S) amino acid sequence:**
   Based on SEQ ID NO: 2, the amino acid E at position 64 is mutated to V and the amino acid N at position 186 is mutated to S.
**M19 (V79M, D374N, Q536R) amino acid sequence:**
   Based on SEQ ID NO: 2, the amino acid V at position 79 is mutated to M, the amino acid D at position 374 is mutated to N and the amino acid Q at position 536 is mutated to R.
**M20 (N231K) amino acid sequence:**

   Based on SEQ ID NO: 2, the amino acid N at position 231 is mutated to K.
**M21 (N231R) amino acid sequence:**

   Based on SEQ ID NO: 2, the amino acid N at position 231 is mutated to R.
**M22 (N231A) amino acid sequence:**

   Based on SEQ ID NO: 2, the amino acid N at position 231 is mutated to A.
**M23 (A229R) amino acid sequence:**

   Based on SEQ ID NO: 2, the amino acid A at position 229 is mutated to R.
**M24 (A229K) amino acid sequence:**
   Based on SEQ ID NO: 2, the amino acid A at position 229 is mutated to K.
**M25 (A229K, N231K) amino acid sequence:**
   Based on SEQ ID NO: 2, the amino acid A at position 229 is mutated to K and the amino acid N at position 231 is mutated to K.
**M26 (A229R, N231K) amino acid sequence:**
   Based on SEQ ID NO: 2, the amino acid A at position 229 is mutated to R and the amino acid N at position 231 is mutated to K.
**M27 (N231K, F293L) amino acid sequence:**
   Based on SEQ ID NO: 2, the amino acid N at position 231 is mutated to K and the amino acid F at position 293 is mutated to L.
**M28 (A229K, N231R) amino acid sequence:**
   Based on SEQ ID NO: 2, the amino acid A at position 229 is mutated to K and the amino acid N at position 231 is mutated to R.
**M29 (A229R, N231R) amino acid sequence:**
   Based on SEQ ID NO: 2, the amino acid A at position 229 is mutated to R and the amino acid N at position 231 is mutated to R.
**M30 (N231R, F293L) amino acid sequence:**
   Based on SEQ ID NO: 2, the amino acid N at position 231 is mutated to R and the amino acid F at position 293 is mutated to L.
**M31 (A229K, N231A) amino acid sequence:**
   Based on SEQ ID NO: 2, the amino acid A at position 229 is mutated to K and the amino acid N at position 231 is mutated to A.
**M32 (A229R, N231A) amino acid sequence:**
   Based on SEQ ID NO: 2, the amino acid A at position 229 is mutated to R and the amino acid N at position 231 is mutated to A.
**M33 (N231A, F293L) amino acid sequence:**
   Based on SEQ ID NO: 2, the amino acid N at position 231 is mutated to A and the amino acid F at position 293 is mutated to L.
**M34 (A229K, N231K, F293L) amino acid sequence:**
   Based on SEQ ID NO: 2, the amino acid A at position 229 is mutated to K, the amino acid N at position 231 is mutated to K and the amino acid F at position 293 is mutated to L.
**M35 (A229R, N231K, F293L) amino acid sequence:**
   Based on SEQ ID NO: 2, the amino acid A at position 229 is mutated to R, the amino acid N at position 231 is mutated to K and the amino acid F at position 293 is mutated to L.
**M36 (A229K, N231R, F293L) amino acid sequence:**
   Based on SEQ ID NO: 2, the amino acid A at position 229 is mutated to K, the amino acid N at position 231 is mutated to R and the amino acid F at position 293 is mutated to L.
**M37 (A229R, N231R, F293L) amino acid sequence:**
   Based on SEQ ID NO: 2, the amino acid A at position 229 is mutated to R, the amino acid N at position 231 is mutated to R and the amino acid F at position 293 is mutated to L.
**M38 (A229K, N231A, F293L) amino acid sequence:**
   Based on SEQ ID NO: 2, the amino acid A at position 229 is mutated to K, the amino acid N at position 231 is mutated to A and the amino acid F at position 293 is mutated to L.

### Example 1: Prediction and overall structural comparison of the three-dimensional structure of firefly (Photuris pensylvanica) luciferase.

The overall spatial structure of the luciferase from *Photuris pensylvanica* was successfully predicted using a protein structure prediction tool. To further explore more useful information to guide in-depth research and analysis of the binding mode between luciferase and its substrate, the predicted structure was aligned and analyzed with the published complex structure of luciferase and its substrate in the PDB database (PDB ID: 5dwv).

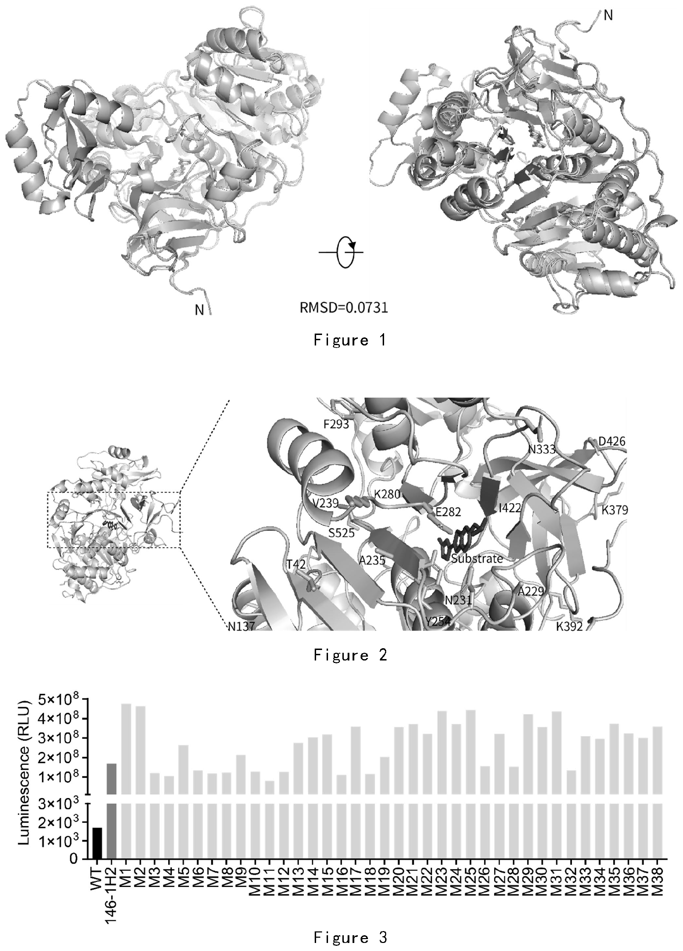

The results are shown in Figure 1: a comparison of the luciferase structure of *Photuris pensylvanica* and the 5dwv structure from two different perspectives. The results indicate that the two structures share the same topological conformation overall, with high structural similarity (RMSD=0.0731), and the spatial regions responsible for substrate binding are consistent.

### Example 2: Analysis of the three-dimensional structure of firefly (Photuris pensylvanica) luciferase and display of its substrate-binding pocket.

Based on the structure, it is known that one luciferase molecule binds to one substrate molecule. The substrate binds to a hydrophobic pocket composed of one α-helix, one loop, and four β-sheets of luciferase. Some amino acids located near this hydrophobic pocket provide effective interaction forces for the anchoring of the substrate through their side chains, participating in stabilizing the specific recognition and binding of the substrate. In addition, amino acids located at the entrance and exit of the substrate-binding pocket can close the pocket with their side chains, jointly preventing the bound substrate from overflowing and releasing.

The results are shown in Figure 2: detailed display of the substrate-binding pocket. On the left is the overall three-dimensional structure of the luciferase from *Photuris pensylvanica.* The local detailed view of the interaction between luciferase and substrate is presented on the right.

### Example 3: Construction of Mutants and Expression of Luciferase

Based on the results of structural analysis, a series of site-directed mutagenesis were designed and carried out on the luciferase. The PCR products of site-directed mutagenesis were digested with Dpn I and then transformed into competent E. coli DH5α cells. Three single colonies were picked and cultured in small quantities, and plasmids were extracted. Sequencing was performed to verify the sequence, and clones successfully undergoing site-directed mutagenesis were obtained.

Finally, all the designed site-directed mutants obtained positive clones, and the plasmid sequences were verified by sequencing to ensure accuracy; whereas the random mutants were obtained by directly screening and sequencing the clones using a random mutation kit.

All 38 firefly luciferase mutants, as well as the control WT and 146-1H2, exhibited significant expression in the supernatant.

### Example 4: Enzyme activity detection of crude enzymes from wild type, 146-1H2, and various mutants

Bac-Lumi^{™} bacterial firefly luciferase detection reagent contains both Escherichia coli cell lysis components and firefly luciferase activity detection components, eliminating the need to collect and lyse cells. It allows for the one-step determination of firefly luciferase enzyme activity in bacteria. In a 96-well plate, 10-fold diluted induced bacterial solution (100µl) was mixed with detection reagent (100µl) at a ratio of 1:1. The mixture was incubated on a horizontal shaker at room temperature for 10 minutes with agitation, followed by chemiluminescence detection using a multifunctional microplate reader. The luminescence signal measured at the 1-hour time point was used for plotting (for WT, which exhibits rapid signal decay, the signal value at the 10-minute mark was taken for plotting).

The results are shown in Figure 3: the signal value of the wild type (WT) is 1.7×10^{^3} RLU, the signal of the control (146-1H2) is approximately 1.7×10^{^8} RLU and the enzyme activity signals of M1-M38 range from 8.1×10^{^7} to 4.8×10^{^8} RLU.

### Example 5: Expression, purification and quantification of firefly luciferase protein with significant effect on crude enzyme activity detection

Based on the results of crude enzyme activity assay, 23 mutants with relatively high enzyme activity were obtained. These mutants, along with the control (WT, 146-1H2), were each expressed in BL21(DE3) strain at 1L scale, and affinity purification was carried out using a Ni affinity chromatography column. The protein concentration of the purified proteins was determined by the Bradford method, diluted to the same concentration of 1 mg/ml with enzyme storage solution, and SDS-PAGE was used to confirm whether the concentrations of 25 luciferase protein samples were the same.

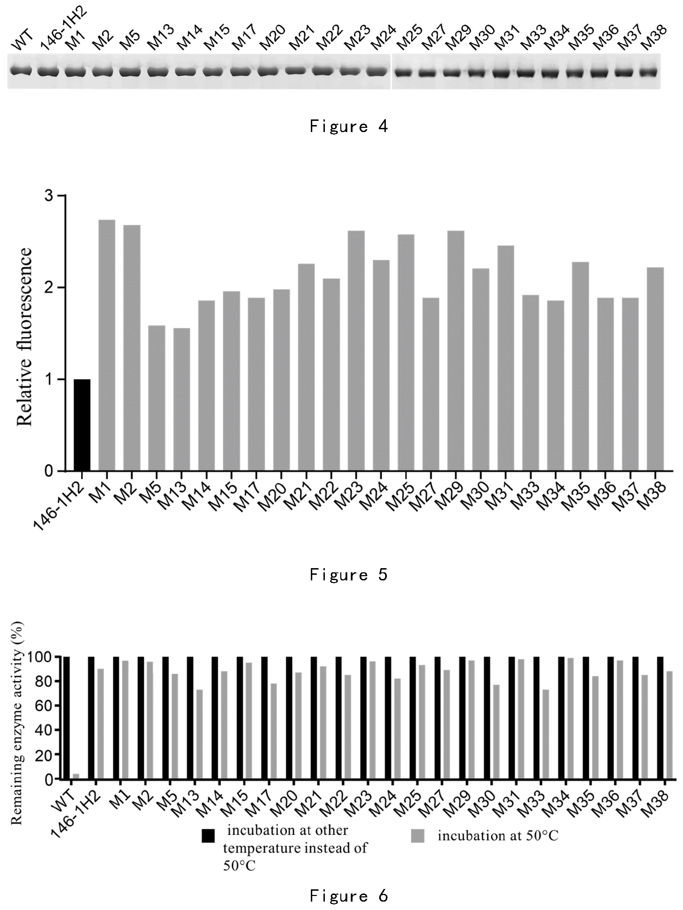

The results are shown in Figure 4: The loading volume of each sample was 1 µg, and the protein content among the samples was basically consistent.

### Example 6: Comparison of enzyme activity detection of purified firefly luciferase

In this example, each luciferase mutant diluted to 1 mg/ml with enzyme storage solution was used for enzyme activity detection.

In a 200µl reaction system, the final protein concentration of various mutants of firefly luciferase and the control (WT, 146-1H2) was 25µg/ml, and the signal values at 10 minutes were measured for plotting.

The results are shown in Figure 5: Compared with the control (146-1H2), the enzyme activities of the remaining 23 firefly luciferase mutants were significantly increased, approximately 1.6-2.8 times higher than that of the control. This enzyme activity detection result is basically consistent with the previous crude enzyme activity detection result.

### Example 7: Comparison of signal thermal stability of purified firefly luciferase

In this example, each luciferase mutant diluted to 1 mg/ml with enzyme storage solution was used for enzyme activity detection.

In a 200 µl reaction system, the final protein concentration of various mutants of firefly luciferase and the control (WT, 146-1H2) was 25 µg/ml. The firefly luciferase protein samples with calibrated protein concentrations were treated in two ways: before and after incubation at 50 °C, followed by enzyme activity detection. Based on the degree of change in enzyme activity before and after incubation at 50 °C, the thermal stability of each luciferase mutant was analyzed.

The results are shown in Figure 6: After incubation at 50°C, the enzyme activity of wild-type luciferase was almost completely lost; the enzyme activity of luciferase mutant 146-1H2 slightly decreased, with a decrease of about 10% compared to before incubation; after incubation at 50°C, the enzyme activity of the remaining luciferase mutants showed varying degrees of decrease ranging from 1% to 27% and all mutants exhibited high or very high thermal stability.

### Example 8: High-enzyme-activity firefly luciferase mutant for quantitative detection of ATP

Based on the preliminary test results, two firefly luciferase mutants, M1 (F293L) and M2 (Y254S), were selected to test whether they could be used for quantitative detection of ATP.

In a 200 µl reaction system, the final protein concentrations of firefly luciferase mutants M1 (F293L), M2 (Y254S) and the control (146-1H2) were 25 µg/ml; the final concentrations of ATP were 0, 0.125, 0.25, 0.5, 1, 1.5, and 2 µM.

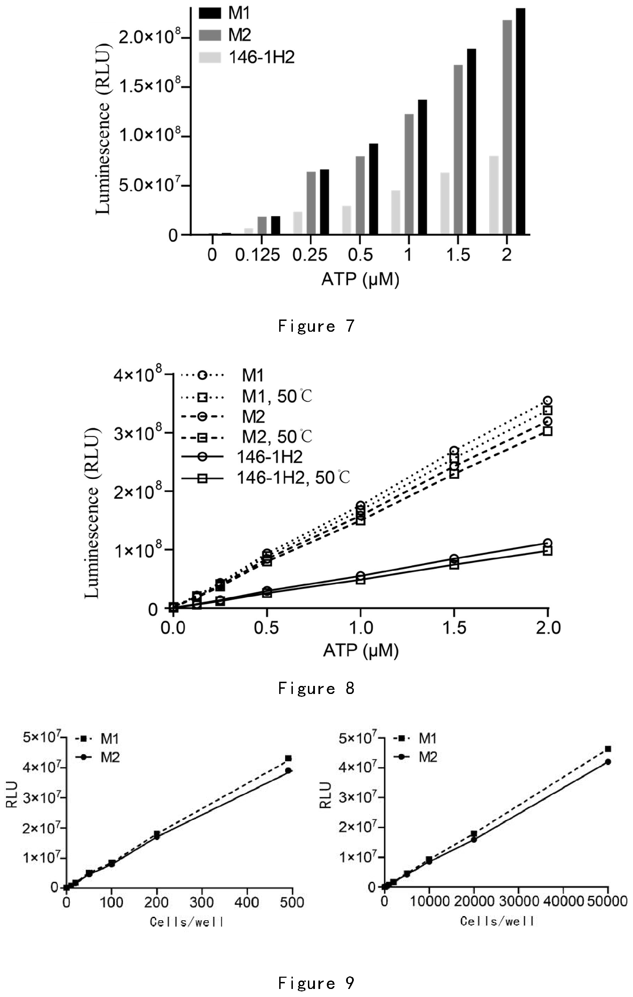

The signal values at 10 minutes were plotted, and the results are shown in Figure 7: The fluorescence signal values of both M1 (F293L) and M2 (Y254S) mutants were significantly higher than that of the control (146-1H2).

The fold change in signal values of the mutants compared to the control at various ATP concentrations (excluding 0 µM) was calculated. The enzyme activity of M1 (F293L) is approximately 2.82 ± 0.09 times higher than that of the control (146-1H2), and the enzyme activity of M2 (Y254S) is approximately 2.72 ± 0.05 times higher than that of the control (146-1H2). In other words, the enzyme activities of mutants M1 (F293L) and M2 (Y254S) are approximately 182% and 172% higher than that of the control (146-1H2), respectively.

### Example 9: The high enzyme activity firefly luciferase mutant remain suitable for quantitative detection of ATP after 50°C heat treatment

Firefly luciferase samples at 1 mg/ml were subjected to heat treatment at 50°C for 24 hours. After centrifugation, the supernatant was collected, and its enzyme activity was compared with that of the non-heat-treated luciferase. The signal values measured at 10 minutes were plotted.

The results are shown in Figure 8: The signal values of all samples were positively correlated with the concentration of ATP, indicating that both mutants M1 (F293L) and M2 (Y254S), like the control (146-1H2), can be used to detect the concentration of ATP in solution. Overall, the enzyme activity of M1 (F293L) is approximately 2.8 times that of the control (146-1H2), while the enzyme activity of M2 (Y254S) is close to 2.7 times that of the control (146-1H2).

The result is consistent with that in Figure 6, indicating good repeatability of the experiment.

By comparing the data of the same protein before and after heat treatment, it was found that treatment at 50°C for 24 hours did not affect the enzymatic activity of the protein, indicating that M1 (F293L) and M2 (Y254S) have good thermal stability.

### Example 10: Comparison of the effects of high-enzyme-activity firefly luciferase mutants in cell enzyme activity detection

The purified high-enzyme activity firefly luciferase mutants M1 and M2 were used to measure the ATP content of different numbers of Hela cells by chemiluminescence assay, reflecting cell viability, thus achieving the purpose of quantitative detection of live cell numbers with ultra-high sensitivity and ultra-wide linear range. Cells were lysed with 1% Triton X-100 for ATP concentration detection. The signal values at 10 minutes of detection were plotted.

The results are shown in Figure 9: The luciferase mutant can be used for cell enzyme activity detection, with high detection sensitivity and a wide linear range. It can exhibit a good linear relationship within a range of at least 0 to 50,000 cells.

### Experimental conclusion

Through structural prediction and comparative analysis, combined with site-directed and random mutagenesis, a series of firefly luciferase mutants were designed and screened based on in-depth analysis and experimental research conducted by the inventor. After preliminary screening, 23 mutants with high enzyme activity were subjected to expression purification and enzyme activity assay.

The assays revealed that, compared to the control (146-1H2), all 23 purified mutants showed significantly improved enzyme activity. After incubation at 50°C for 24 hours, all 23 mutants retained a relatively high level of enzyme activity. Among them, M1 (F293L) and M2 (Y254S) exhibited relatively better enzyme activity and thermal stability. Using these two enzyme mutants as examples, it was found that they perform well for ATP quantitation. Furthermore, these mutants remained fully functional for ATP quantitation even after incubation at 50°C for 24 hours. Furthermore, such mutants were also found to be used for detecting cell number or cell viability.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A method for enhancing enzyme activity and/or thermal stability of a firefly luciferase, comprising: mutating and modifying the firefly luciferase to form a firefly luciferase mutant, wherein the mutant has a spatial structure adjacent to the substrate-binding pocket, contributing to the recognition and binding of the substrate or the stability of the protein's spatial conformation.

2. The method according to claim 1, wherein the mutant is a protein comprising the following mutation(s) corresponding to the amino acid sequence shown in SEQ ID NO: 2:
A. mutation(s) selected from (a)-(t) or a combination thereof:
(a) a mutation of the amino acid F at position 293 to L (M1);
(b) a mutation of the amino acid Y at position 254 to S (M2);
(c) a mutation of the amino acid V at position 239 to A (M5);
(d) a mutation of the amino acid N at position 137 to K, a mutation of the amino acid A at position 235 to T, a mutation of the amino acid K at position 280 to R, a mutation of the amino acid N at position 333 to D, and a mutation of the amino acid I at position 422 to V (M13);
(e) a mutation of the amino acid K at position 57 to R (M14);
(f) a mutation of the amino acid T at position 42 to A, a mutation of the amino acid E at position 282 to D, a mutation of the amino acid K at position 392 to I, and a mutation of the amino acid D at position 426 to V (M15);
(g) a mutation of the amino acid S at position 525 to P (M17);
(h) a mutation of the amino acid N at position 231 to A, R or K (M20, M21, or M22);
(i) a mutation of the amino acid A at position 229 to R or K (M23 or M24);
(j) a mutation of the amino acid A at position 229 to K and a mutation of the amino acid N at position 231 to K (M25);
(k) a mutation of the amino acid N at position 231 to K and a mutation of the amino acid F at position 293 to L (M27);
(l) a mutation of the amino acid A at position 229 to R and a mutation of the amino acid N at position 231 to R (M29);
(m) a mutation of the amino acid N at position 231 to R and a mutation of the amino acid F at position 293 to L (M30);
(n) a mutation of the amino acid A at position 229 to K and a mutation of the amino acid N at position 231 to A (M31);
(o) a mutation of the amino acid N at position 231 to A and a mutation of the amino acid F at position 293 to L (M33);
(p) a mutation of the amino acid A at position 229 to K, a mutation of the amino acid N at position 231 to K, and a mutation of the amino acid F at position 293 to L (M34);
(q) a mutation of the amino acid A at position 229 to R, a mutation of the amino acid N at position 231 to K, and a mutation of the amino acid F at position 293 to L (M35);
(r) a mutation of the amino acid A at position 229 to K, a mutation of the amino acid N at position 231 to R, and a mutation of the amino acid F at position 293 to L (M36);
(s) a mutation of the amino acid A at position 229 to R, a mutation of the amino acid N at position 231 to R, and a mutation of the amino acid F at position 293 to L (M37);
(t) a mutation of the amino acid A at position 229 to K, a mutation of the amino acid N at position 231 to K, and a mutation of the amino acid F at position 293 to L (M38);
B. a protein derived from the protein in A and formed by substituting, deleting or adding one or more amino acid residues in the amino acid sequence of the protein in A with the function of the protein in A, but with conserved positions corresponding to (a)-(t) defined in A;
C. a protein derived from the protein in A with more than 80% homology with the amino acid sequence of the protein in A and the function of the protein in A, but with conserved positions corresponding to (a)-(t) defined in A.

3. A firefly luciferase mutant, wherein the mutant has a spatial structure adjacent to the substrate-binding pocket, contributing to the recognition and binding of the substrate or the stability of the protein's spatial conformation, and the mutant is a protein comprising the following mutation(s) corresponding to the amino acid sequence shown in SEQ ID NO: 2:
A. mutation(s) selected from (a)-(t) or a combination thereof:
(a) a mutation of the amino acid F at position 293 to L (M1);
(b) a mutation of the amino acid Y at position 254 to S (M2);
(c) a mutation of the amino acid V at position 239 to A (M5);
(d) a mutation of the amino acid N at position 137 to K, a mutation of the amino acid A at position 235 to T, a mutation of the amino acid K at position 280 to R, a mutation of the amino acid N at position 333 to D, and a mutation of the amino acid I at position 422 to V (M13);
(e) a mutation of the amino acid K at position 57 to R (M14);
(f) a mutation of the amino acid T at position 42 to A, a mutation of the amino acid E at position 282 to D, a mutation of the amino acid K at position 392 to I, and a mutation of the amino acid D at position 426 to V (M15);
(g) a mutation of the amino acid S at position 525 to P (M17);
(h) a mutation of the amino acid N at position 231 to A, R or K (M20, M21, or M22);
(i) a mutation of the amino acid A at position 229 to R or K (M23 or M24);
(j) a mutation of the amino acid A at position 229 to K and a mutation of the amino acid N at position 231 to K (M25);
(k) a mutation of the amino acid N at position 231 to K and a mutation of the amino acid F at position 293 to L (M27);
(l) a mutation of the amino acid A at position 229 to R and a mutation of the amino acid N at position 231 to R (M29);
(m) a mutation of the amino acid N at position 231 to R and a mutation of the amino acid F at position 293 to L (M30);
(n) a mutation of the amino acid A at position 229 to K and a mutation of the amino acid N at position 231 to A (M31);
(o) a mutation of the amino acid N at position 231 to A and a mutation of the amino acid F at position 293 to L (M33);
(p) a mutation of the amino acid A at position 229 to K, a mutation of the amino acid N at position 231 to K, and a mutation of the amino acid F at position 293 to L (M34);
(q) a mutation of the amino acid A at position 229 to R, a mutation of the amino acid N at position 231 to K, and a mutation of the amino acid F at position 293 to L (M35);
(r) a mutation of the amino acid A at position 229 to K, a mutation of the amino acid N at position 231 to R, and a mutation of the amino acid F at position 293 to L (M36);
(s) a mutation of the amino acid A at position 229 to R, a mutation of the amino acid N at position 231 to R, and a mutation of the amino acid F at position 293 to L (M37);
(t) a mutation of the amino acid A at position 229 to K, a mutation of the amino acid N at position 231 to K, and a mutation of the amino acid F at position 293 to L (M38);
B. a protein derived from the protein in A and formed by substituting, deleting or adding one or more amino acid residues in the amino acid sequence of the protein in A with the function of the protein in A, but with conserved positions corresponding to (a)-(t) defined in A;
C. a protein derived from the protein in A with more than 80% homology with the amino acid sequence of the protein in A and the function of the protein in A, but with conserved positions corresponding to (a)-(t) defined in A.

4. A polynucleotide, wherein the polynucleotide encodes the firefly luciferase mutant according to claim 3.

5. A vector or a genetically engineered host cell comprising the vector, wherein the vector comprises the polynucleotide according to claim 4; or the host cell comprises the vector or has the polynucleotide according to claim 4 integrated into its genome.

6. A method for producing a firefly luciferase mutant according to claim 3, wherein the method comprises the following steps:
(i) culturing the host cell according to claim 5;
(ii) collecting a culture comprising the firefly luciferase mutant;
(iii) isolating the firefly luciferase mutant from the culture.

7. A use of the firefly luciferase mutant according to claim 3, the host cell expressing the mutant or a lysate thereof, for catalyzing the oxidation of firefly luciferase substrate and generating ATP-dependent bioluminescence, or for detecting cell number or cell viability.

8. A method for catalytically oxidizing firefly luciferin to produce bioluminescence, comprising: catalytically oxidizing using the firefly luciferase mutant according to claim 3, a host cell expressing the mutant or a lysate thereof, and producing bioluminescence in the presence of ATP.

9. The method according to claim 8, wherein the method is used for reactions comprising the following groups: in vitro ATP detection, cell viability detection and cell number detection.

10. The method according to claim 9, wherein the in vitro ATP detection comprises: using the firefly luciferase mutant and luciferase substrate to measure the amount of ATP comprised in the solution system; or
the cell viability detection comprises mixing the firefly luciferase mutant and luciferase substrate with cell cultures to measure cell viability; or
the cell number detection comprises mixing the firefly luciferase mutant and luciferase substrate with cell cultures to measure the cell number.

11. A detection system or detection kit for catalyzing the oxidation of firefly luciferin to produce bioluminescence, comprising: the firefly luciferase mutant according to claim 3, or the host cell according to claim 5, a culture thereof or a lysate thereof.

12. An ATP tester, comprising:
a container comprising the firefly luciferase mutant according to claim 3;
a container comprising a luciferase substrate within it;
sampling, dispensing and reaction devices used for adding a sample to be tested, wherein the sample to be tested is a sample to be tested by ATP detection.

13. The ATP tester according to claim 12, wherein it comprises a gas supply device, wherein the gas comprises oxygen.
